# EUROPEAN PATENT SPECIFICATION

(11) **EP 0 870 045 B1**
(45) Date of publication and mention of the grant of the patent: **12.07.2006**
(21) Application number: 96943232.7
(22) Date of filing: 23.12.1996
(51) Int. Cl.: C12N 15/85, C12N 5/10, A01K 67/027, C12N 15/53

(54) **GENE CONTROL**
KONTROLLE DER GENEXPRESSION
REGULATION GENIQUE

(30) Priority: 23.12.1995 GB 9526509
(43) Date of publication of application: 14.10.1998
(73) Proprietor: THE UNIVERSITY COURT OF THE UNIVERSITY OF DUNDEE, Dundee DD1 4HN, Scotland (GB); ROSLIN INSTITUTE (EDINBURGH), Roslin, Midlothian EH25 9PS (GB)
(72) Inventor: CLARK, Anthony, John, Lasswade, Midlothian EH18 1LN (GB); WOLF, Charles, Roland, Inchture PH14 9QS (GB)
(74) Representative: Main, Malcolm Charles
(86) International application number: PCT/GB1996/003212
(87) International publication number: WO 1997/023635

(56) References cited:
- WO-A-94/17208
- WO-A-94/29442
- WO-A-96/01313
- WO-A-96/40892
- MOLECULAR PHARMACOLOGY, vol. 44, no. 3, September 1993, WILLIAMS & WILKINS, NY, US, pages 560-568, XP000670033 K. STERLING ET AL.: "Rat CYP1A1 negative regulatory element: Biological activity and interaction with a protein from liver and hepatoma cells"
- CARCINOGENESIS, vol. 9, no. 9, September 1988, IRL PRESS LIMITED, OXFORD, ENGLAND, pages 1599-1605, XP000670064 R.N. HINES ET AL.: "Identification of multiple regulatory elements on the human cytochrome P450IaI gene"
- PROC. NATL.ACAD SCI., vol. 92, no. 25, 5 December 1995, NATL. ACAD SCI.,WASHINGTON,DC,US;, pages 11926-11930, XP002029019 J.S. SMITH ET AL.: "Cytochrome P450 1A1 promoter as a genetic switch for the regulatable and physiological expression of a plasma protein in transgenic mice"
- J. CLIN. INVEST., vol. 93, no. 4, April 1994, ROCKEFELLER UNIVERSITY PRESS, NEW YORK, US, pages 1683-1690, XP000670068 N.S. SHACHTER ET AL.: "Overexpression of apolipoprotein CII causes hypertriglyceridemia in transgenic mice"
- NUCLEIC ACIDS RESEARCH, vol. 19, no. 23, 11 December 1991, IRL PRESS LIMITED,OXFORD,ENGLAND, pages 6547-6551, XP002029020 S.N. JONES ET AL.: "Induction of the Cyp1a-1 dioxin enhancer in transgenic mice" cited in the application
- PROC. NATL.ACAD SCI., vol. 83, no. 21, November 1986, NATL. ACAD SCI.,WASHINGTON,DC,US;, pages 8044-8048, XP002029021 K. SOGAWA ET AL.: "Location of regulatory elements responsible for drug induction in the rat cytochrome P-450c gene" cited in the application
- INT. J. BIOCHEM., vol. 21, no. 3, 1989, PERGAMON PRESS PLC, GREAT BRITAIN, pages 243-252, XP000647926 D.W. NEBERT AND J.E. JONES: "Regulation of the mammalian cytochrome P1-450 (CYP1A1) gene"
- J. CELL SCIENCE, vol. 109, no. 11, November 1996, THE COMPANY OF BIOLOGISTS LIMITED, GB, pages 2619-2625, XP000670031 S.J. CAMPBELL ET AL.: "Regulation of the CYP1A1 promoter in transgenic mice: an exquisitely sensitive on-off system for cell specific gene regulation"

## Description

The present invention is concerned with controlling the expression of genetic material and is particularly concerned with the expression control of transferred genetic material in vivo.

Genetic engineering has been recognised as a powerful technique not only in research but also for commercial purposes. Thus, by using genetic engineering techniques exogenous genetic material can be transferred to a host cell and the protein or polypeptide encoded by the exogenous genetic material may be expressed within the host. For the purposes of simplicity genetic engineering is normally carried out with prokaryotic microorganisms, for example bacteria such as E. coli. However, use has also been made of eukaryotic systems, in particular simple microorganisms such as yeast or algae, although for certain applications eukaryotic cell cultures have also been used.

More recently transgenic animals have been produced. Here the foreign genetic material has been introduced into the fertilized egg of an animal which then proceeds to develop into an embryo in the normal manner having been transplanted into a foster mother. Truly transgenic animals contain copies of the exogenous DNA in each cell. It is also possible to produce so called "mosaic" animals in which the foreign DNA is only contained in specific tissues. Mosaic animals are generally produced by introducing exogenous DNA into an embryo at a later developmental stage.

In the production of transgenic animals a high copy number of the foreign genetic material of interest is injected into the fertilized stem cell. Multiple copies of the transgene are destroyed in the host cell, but hopefully one or more copies undergo random integration into the endogenous chromosomal DNA. To date it has not been possible to control the location at which the transgene integrates into the host chromosome merely by microinjection.

Embryonic stem cells (ES cells) are also a potential means of introducing foreign genetic material into the germline.

Normally the genetic material to be transferred, whether to a transgenic animal or simply to transform a single cell, is flanked by expression control sequences.

The term "expression control sequence" is used herein to refer to any sequence of genetic material which ultimately affects the expression of the protein or polypeptide encoded thereby. Thus, the term "expression control sequence" includes promoters, enhancers, repressors and other regulators which may be upstream or downstream of the protein encoding sequence of interest. It is also possible for the expression control sequence to be contained within the protein encoding sequence itself, or to be within an intron (non-coding portion) interrupting the protein encoding sequence.

Such expression control sequences must generally be included to ensure that the foreign protein or polypeptide is successfully expressed in the transformed host cell and these can be part of an endogenous promoter or engineered from regulatory elements.

However, in many situations it is not desirable for the expression of the protein or polypeptide of interest to be constitutive, that is to say it is not desirable for the protein or polypeptide of interest to be continually expressed. This is especially a problem in transgenic animals where expression of the foreign protein will not be required in all tissues or if a gene is lethal if continuously expressed or creates unknown effects or phenotypes.

Previous attempts to resolve this problem for transgenic animals includes the use of expression control sequences which are native to the host animal and are only active in specific tissues. Mention may be made of the β-lactoglobulin promoter which when linked to the foreign protein encoding sequence of interest causes expression of the protein or polypeptide encoded thereby in the mammary glands only.

Another possibility is to use an expression control sequence which is inducible so that expression of the transgene may be controlled by exogenous factors. One well known inducible promoter is the metallothionein promoter which becomes active in the presence of heavy metals such as Zn²⁺ or Cd²⁺. However, this promoter has the disadvantage that it exhibits significant levels of basal expression in many tissues as discussed by Karin et al in Cell 36:371-379 (1984). Likewise hormones control the expression of a wide variety of genetic material but the biological hormonal side-effects exhibited render them unsuitable candidates for selective inducing agents.

Unlike many of the prokaryotic expression control systems most eukaryotic promoters cannot be regulated by external control in a straightforward manner. Therefore the on/off regulation of transgenes has not so far been achieved.

It has recently been found that the promoter elements of genes expressing a drug metabolising enzyme are useful for the regulated control of gene expression in transgenic animals.

In 1991, Jones et al (Nucleic Acid Research Vol. 19 No. 23 6547-6551) reported the induction of expression of a reporter gene by the CYP1A1 dioxin-responsive promoter in transgenic mice.

The CYP1A1, whose product, aryl hydrocarbon hydroxylase assists in detoxification of polycyclic aromatic hydrocarbons, is the best characterized of the murine cytochrome P450 genes. The CYP1A1 dioxin-responsive enhancer region has been previously analysed in vitro and found to induce expression of heterologous genes upon exposure of transfected cells to various aromatic hydrocarbons. A 2.58 kbp DNA fragment containing the CYP1A1 enhancer elements and promoter region was coupled to the chloramphenicol acetyltransferase (CAT) reporter gene and used to create transgenic mice. CAT assays were performed on tissues harvested from three different lines of transgenic mice following mock-induction or induction using the aromatic hydrocarbon, 3-methylcholanthrene. Basal levels of expression were detected in the spleen and small bowel of non-induced mice, with little or no expression detected in the liver. Treatment with 3-methylcholanthrene increased hepatic expression levels by as much as 10,000-fold. More modest levels of induction were also recorded in the spleen, small bowel, kidney and lung. The results indicate that the dioxin-responsive enhancer region functions as a strongly inducible promoter in vivo. Differences in the response to induction between male and female mice suggest that CYP1A1 expression may be governed in a gender related manner.

Whereas Jones et al. show substantial induction of CAT in the liver of transgenic mice, they also report that there are still significant levels of basal expression in this tissue. Their construct therefore shows little advantage over the aforementioned metallothionine promoter and their teaching suggests that the CYP1A1 promoter cannot be used as a truly "on/off switch" to regulate transgenic expression.

Also, the 2.58 kb mouse promoter confers substantial levels of basal CAT expression in the spleen and the bowel and little induction is seen. Further, Jones et al. show very modest induction in the kidney and lung and in their paper they conclude that the mouse CYP1A1 dioxin responsive enhancer region is likely to have utility as an inducible regulator of "hepatic transgene expression" only. Their results do not suggest that it would be useful in other tissues and they clearly do not anticipate its use as an inducible regulator of expression in any tissues other than the liver. Nor do they anticipate the use of this promoter to achieve "on/off" regulation in the liver.

Other publications (Schacter et al, 1994 and Smith et al, 1995) also relating to CYP1A1 promoters to drive transgene expression in mice have also demonstrated the potential of the model for regulating hepatic gene expression. However, as with Jones et al, variable background levels and inconsistent tissue specific expression were observed.

It is an object of the present invention to achieve "on/off" transgene regulation in a wide variety of tissues.

The present invention thus provides a recombinant genetic material according to claim 1. The protein encoding sequence is operably linked to an expression control sequence of gene regulated by drugs or a xenobiotic compound, such as a drug metabolising enzyme. The expression control sequence comprises at least one promoter region and at least one repressor region wherein the at least one repressor region significantly reduces basal expression at least in a tissue where the protein is required to be expressed whilst allowing the induction of expression of the protein encoding sequence in a manner similar to which the gene is regulated in vivo.

The promoter region is derived from could be the CYP1A1 promoter.

The invention provides plasmid pAHIR1β-gal deposited with the National Collections of Industrial and Marine Bacteria Limited in Aberdeen, UK on 23 December 1996 under accession number 40851.

The drug metabolising enzymes themselves have evolved as an adaptive response system against the toxic challenge of environmental chemicals. As part of this response, exposure to a particular chemical generally induces the expression of specific proteins active in the metabolism of that chemical, often in a tissue specific manner. The basal level of expression is extremely low or negligible and the induction of gene expression can be significant.

The regulatory sequences of any drug metabolising enzyme may be suitable and mention may be made of the cytochrome P450-dependent mono-oxygenase and the glutathione S-transferase as examples. However, the present invention is not specifically limited to these two particular enzymes.

As an example, it is known that several cytochrome P450s of glutathione S-transferase can be induced from an undetectable level to a very high level in the liver and in some cases in other tissues by a diverse range of chemicals. The best characterised promoter of such enzymes is the regulation through a promoter termed the xenobiotic regulatory element (hereinafter XRE). Activation of gene expression by this promoter causes the interruption of the chemical to be inactivated with a cytosolic aryl hydrocarbon (Ah) receptor. The Ah protein then binds to the XRE sequence to activate transcription as described by Nebert et al in Int. J. Biochem 21:243-252 (1989). Induction of the cytochrome P450 CYP1A1 protein occurs by the Ah receptor and is highly conserved in evolution. Promoter systems involving this mechanism of regulation could be used to cause induction of transgene expression.

CYP1A1 transcription is known to be activated by a wide range of compounds in a specific manner. Examples of compounds which cause CYP1A1 transcription include polycyclic aromatic hydrocarbons (PAH), tetrachlorodibenzodioxin (TCDD), beta-naphthoflavone (beta NF) and polychlorinated biphenyls (PCBs). The duration and intensity of the inductive effect will vary according to the precise chemical used and be dependent on its ability to interact with the recept or as well as its pharmacokinetic properties.

The present invention envisages the use of naturally occurring expression control sequences of drug metabolising enzymes but also covers functional equivalents of such sequences and synthetically manufactured versions thereof.

The expression control sequence of the CYP1A1 gene is believed to contain several XRE sequences in a region 3 kilobases upstream from the transcription start site (see Sogawa et al in PNAS USA 83:8044-8048 (1986). This expression control sequence also contains repressor motifs in the region between 650-900 bp and further upstream which may cause the low basal activity of the promoter.

The repressor elements are believed to be absent in the mouse construct described by Jones et al.

In one embodiment therefore the present invention thus provides recombinant genetic material comprising a protein encoding sequence operably linked to an expression control sequence which comprises an XRE. It may be preferable for several XREs, for example 4 to 8 XREs, to be aligned in tandem. Optionally the XRE(s) may be operably linked to a repressor sequence.

The expression control sequence according to the present invention is derived from the P450 CYP1A1 promoter of the rat.

The expression control sequence may be the naturally occurring promoter or an engineered version.

A method of manufacturing of recombinant construct, may comprise forming an operable linkage between an expression control sequence of a drug metabolising enzyme, or functional equivalent thereof, and a protein encoding sequence.

The recombinant genetic material of the present invention may be incorporated into a vector, for example a transfer vector or expression vector. Such vector also form a further aspect of the present invention.

Host cells transfected with a recombinant genetic material according to the present invention, whether in the form of naked DNA or whether in the form of a transfer vector, also form a further aspect of the present invention.

Transgenic animals carrying in their genome (or at least some of their cells) recombinant genetic material as described above form a yet further aspect of the present invention.

The present invention also provides a method of manufacturing proteins or polypeptides, said method comprising the addition of an agent to a cell transformed with a recombinant construct of the invention, whereby to cause the induction of transcription of the protein encoding portion of said construct. The exact nature of the agent to be added will vary depending upon the exact nature of the expression control sequence within the construct. However, particular mention may be made of PAH, TCDD, beta NF and PCBs and 3-mc.

It has been surprisingly found that in contrast to the studies of Jones et al that using the 9 kb rat CYP1A1 promoter linked to a lacZ reporter gene there is no detectable basal expression of lacZ in the liver of transgenic mice but massive induction of expression using 3-methyl-cholanthene (3-mc). The rat promoter thereby acts as an "on/off switch" in the liver.

Presence of repressor sequences in the 9.0 kb rat CYP1A1 segment as opposed to the 2.5 kb segment exemplified by Jones et al probably explains this. This is probably due to the presence of repressor sequences upstream of the promoter (Bouchar, Piechocki and Hines (1995), Molecular and Cellular Biology, pp 5144-5151).

Also, the rat promoter shows no basal expression in the spleen and bowel but demonstrates substantial induction with 3-mc.

The presence of repressor sequences in the 9 kb rat CYP1A1 fragment as opposed to the 2.5 kb fragment of Jones et al probably explains this; as it is probably due to the presence of repressor sequences upstream of the promoter.

Further, using the 9.0 kb rat promoter substantial induction can be achieved in the kidney and lung and again there is no basal expression. In the kidney the expression is restricted to a specific population of cells in the cortex.

Using a 9.0 kb rat expression control sequence in recombinant genetic material according to the present invention inducible expression can be obtained by a wide variety of tissues including liver, oesophagus, jejunum, duodenum, colon, kidney, cortex, adrenal gland, tests, lung and spleen. Skeletal and heart muscle do not exhibit expression either before or after induction.

The profile of expression is thus very akin to the pattern of induction/expression of the endogenous gene.

A further aspect of the present invention provides a method of controlling the expression of a protein encoding sequence, said method comprising the following steps:-
a) Linking said protein encoding sequence to an expression control sequence of the invention in an operable manner;
b) Addition of an agent that interacts with said expression control sequence whereby expression of said protein is enhanced.

The potential of the present invention will now be further described with reference to the following example.

The lacZ gene is used as reporter for these studies. This provides an exquisitely sensitive method to establish tissue and cellular specificity of the promoter and genetically engineered variants and using fluorescent substrates, also enables accurate quantitative measurements of the levels of expression in tissue extracts (7). This can be achieved using lacZ or CAT/promoter constructs. Once the transgenic animals have been generated, work focuses on establishing the ability to regulate promoter activity. Basal, as well as inducible, activity is studied at all stages of development and in all tissues. Regulation of promoter activity using a range of model compounds known to induce or suppress the expression of CYP1A1 is tested. These compounds will be 6-naphthoflavone, polychlorinated biphenyls and tetrachlorodibenzodioxin. The potency and longevity of induction response in a variety of tissues can be established.

In addition to the use of this promoter as a general regulatable system for the expression of transgenes this strategy can be exploited to obtain expression targeted to specific cell types. Thus, for example, when the transgene is incorporated into cells containing a modified hydrocarbon receptor, the inducability of the transgene is modulated. This is exemplified by demonstrating that in DBA mice, which contain a modified Ah receptor, the pattern of induction of the transgene on exposure to inducing agents is changed. For example, 3-methylcholanthrene, which is an inducer of the transgene in C57 BL6 mice, is not an inducer of the transgene in DBA mice. In addition, the introduction of an Ah receptor responsive to 3-methylcholanthrene into a specific cell, e.g. through the use of a tissue-specific promoter element, will allow tissue-specific induction of a transgene in this mouse on administration of 3-methylcholanthrene. This strategy exploits the fact that the Ah receptor in DBA mice through mutation is non-responsive to certain classes of agents which induce by this mechanism. However, in DBA mice induction can still be obtained by certain other types of agents, e.g. by tetrachlorodibenzodioxin. Therefore, using a combination of different forms of the Ah receptor with different types of inducing agent will allow much more precise regulation of transgene expression in cells.

In addition to the up-regulation of this receptor with inducing agents, it is also possible to modulate its activity with the use of compounds which can act as antagonists, i.e. down-regulators such as dibenzofuran. This would allow the extinction of promoter activity at precise time points after activation by the administration of an antagonist. An antagonist would also prevent any opportunistic expression of the promoter due to uncontrolled environmental factors.

The following non limiting examples illustrate the use of the rat CYP1A1 promoter with the aid of the figures wherein:
Figures 1(a) to (d) illustrate generation of pAhIR1β-gal construct;
Figures 2(a) and (b) illustrate Southern Blots to identify transgenic individuals; and
Figure 3 illustrates expression in the liver.

### EXAMPLE 1

### Generation of pAhIR1β-gal

This plasmid was constructed from DNA segments derived from four separate plasmids. The construct comprises ^{~}9kb of the immediate 5' flanking regions, exon 1, intron 1 and part exon II of the rat CYP1A1 gene fused to the lacZ reporter gene. It was designated by pAhIR1β-gal.
1. pA1, comprising 5' flanking regions of the rat CYP1A1 gene: supplied by R. Hines, Dept. of Pharmacology, University of Detroit, Detroit, USA (1).
2. pA8, comprising 5' flanking genomic sequences of the rat cyp1a-1 gene: supplied by R. Hines, Dept. of Pharmacology, University of Detroit, Detroit, USA (1).
Gene accession numbers: GB-RO.RATCYP450(M26129)-537>+999 V00290.
3. pB9, promoterless CAT vector: supplied by CBA Whitelaw(2).
4. bJ6βgal, β-galactosidase reporter gene: supplied by K. Flint, ICRF, London.
1. Foldes, R.L. et al., Arch Biochem Biophys 239, 137-146 (1985).
2. Lang, J.C. et al., Oncogene 6 2067-2075 (1991).

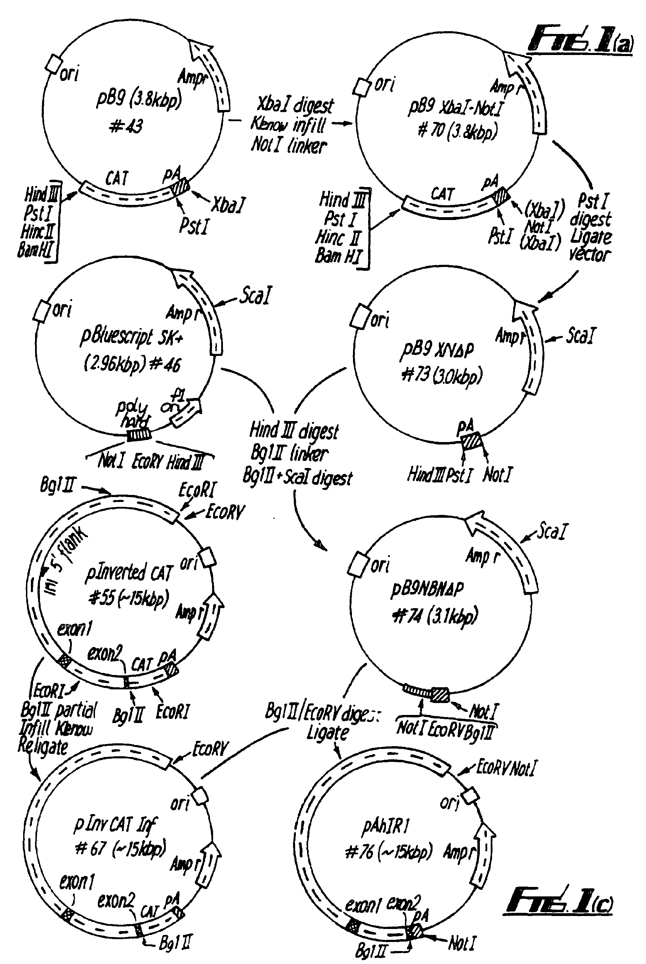

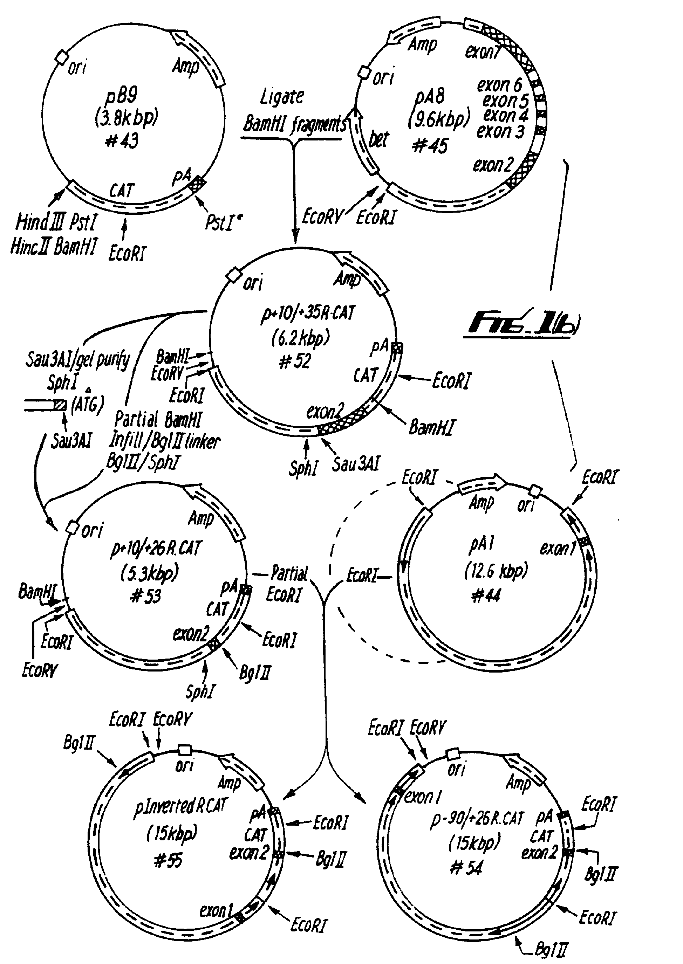

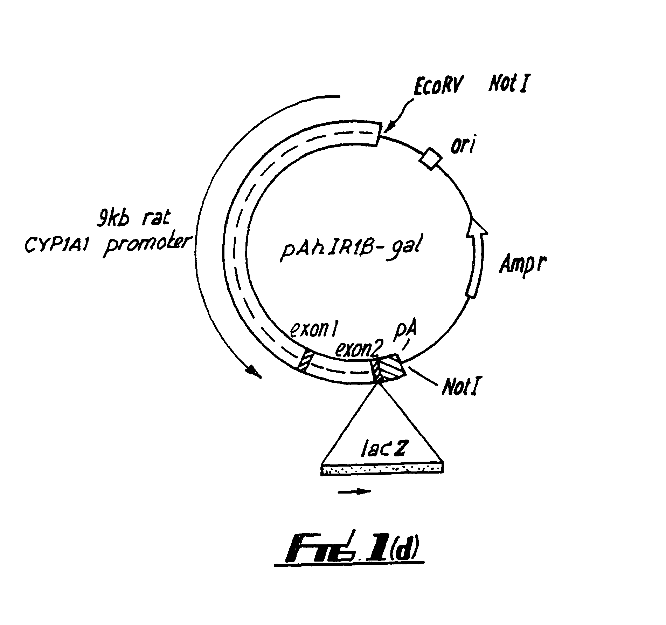

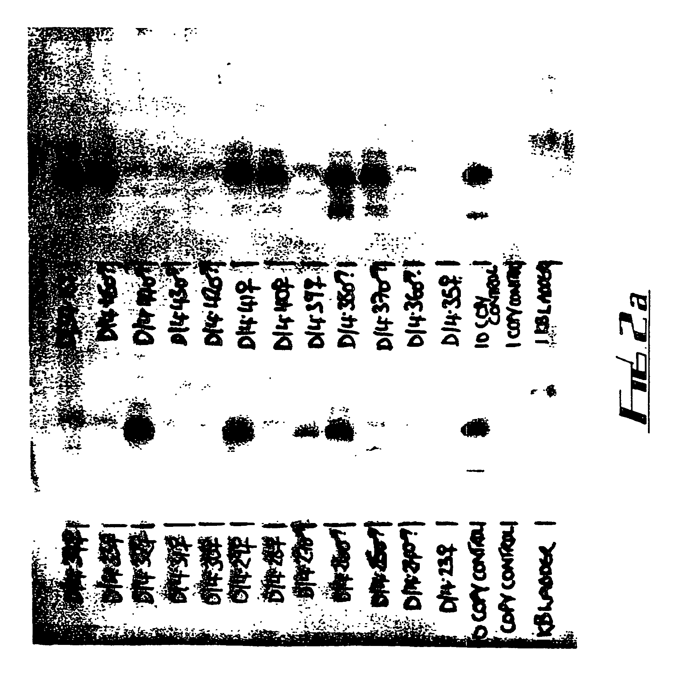

a) Generation of pB9/NBNΔP. Figure 1a the unique XbaI site in pB9 was converted to a NotI site using NotI linkers. The segment of DNA containing the CAT gene was removed by PstI digestion and relegation. The unique HindIII site in this plasmid was converted to a BglII site using BglII linkers. Likewise the unique Hind III site present in the multiple cloning site (MCS) of the commercially available plasmid pBluescript SK+ (Stratagene, Cambs) was converted to a BglII site. The BglII/Scal fragment from each of these plasmids comprising the 5' DNA sequences of the Ampr gene was exchanged. The resulting plasmid pB9/NBNΔP comprises the HSV2IESpA sequences (2) from pB9 linked to the MCS of pBluescript SK+ at a unique BglII site.
b) Generation of pInvertedRCAT. Figure 1b, plasmid pA8 was restricted with BamHI to yield a 2.8 kb fragment containing part of intronl and 2 and exon 2 of the CYP1A1 gene. This plasmid, p+10/+35RCAT was cut with SphI and Sau3A1 and the short fragment comprising part intron 1 and part exon 2 was gel purified. A second batch of p+10/+35RCAT was partially cleaved with BamHI, and the BamHI site in intron 2 of the CYP1A1 gene converted to BglII using BglII linkers. This preparation was cleaved with SphI and BglII and then ligated to the short SphI-Sau3AI fragment isolated above to generate the plasmid p+10/+26RCAT. This plasmid was partially cleaved with EcoRI and the ~9.7kb EcoRI fragment from pA1 (comprising the 5' flanking sequences and exon 1 of the CYP1A1 gene) inserted at the EcoRI site at the 5' end of intron 1 to generate pInvertedRCAT which contains the 5' flanking region, exon 1, intron 1 and part exon 2 of the CYP1A1 gene fused to CAT and HSVIESpA sequence from pB9.
c) Generation of pAhIR1. Figure 1c, plasmid pInvertedCAT was partially cleaved with Bg1II, infilled and relegated to destroy the BglII site present in the 5' flanking regions of the CYP1A1 gene. This leaves a unique BglII site present at the 3' end of exon 2 of the CYP1A1 gene. This plasmid was cleaved with BglII and EcoRV and the fragment containing the CYP1A1 sequences ligated to BglII/EcoRV cleaved pB9NBNΔP to generate pAhIRI. This plasmid comprises ~ 9kb of 5' flanking sequences, exon 1 (untranslated) intron 1 and part exon 2 (untranslated) from the rat CYP1A1 gene linked to the HSVIESpA sequences at a unique BglII site which can be used to insert the desired sequences for expression under the control of the rat CYP1A1 regulatory sequences.
d) Generation of pAhIR1-βgal. Figure 1d the β-galactosidase gene was excised as a ~3kb BamHI fragment from pJ6βgal and inserted at the unique BglII site of pAhIR1. The correct orientation for this fragment was determined by restriction enzyme analysis and sequencing of the BamHI/BglII junctions. The ~18kb insert comprising the CYP1A1 and lacZ sequences was excised from the Not1- cleaved plasmid, purified and prepared for microinjection.

### EXAMPLE 2

### Preparation of DNA for Micro-injection/Production of Transgenic Mice

Plasmid DNA was prepared as described above and checked by digestion with suitable restriction enzymes. The plasmid DNA was digested with NotI, electrophoresed on a 1% agorose gel containing 0.5 mcg/ml ethidium bromide (Sigma). The relevant NotI fragment was located by illumination with a UV lamp (Ultra-Violet Products, Inc., San Gabriel, California, USA). A piece of dialysis membrane was inserted in front of the band and the DNA was subsequently electrophoresed onto the membrane. The DNA was eluted from the dialysis membrane and isolated by use of an "Elu-tip" [Schleicher and Schull, Postfach 4, D-3354, Dassel, W. Germany], employing the procedure recommended by the manufacturer. The isolator DNA fragment was quantified on an agorose/ethidium gel by reference to known amounts of a lambda DNA marker.

### Construction of Transgenic Mice

Procedures are similar to those described by Hogan, Constantini and Lacy in "Manipulating the Mouse Embryo: A Laboratory Manual" Cold Spring Harbor Laboratory (1986).

### Collection of fertilised eggs

Mice used for the collection of fertilised eggs are F₁ hybrids between the C57BL/6 and CBA inbred strains of mice. C67BL/6 females and CBA males are obtained from Harlan Olac Ltd (Shaw's Farm, Bicester, OX6 OTP, England) and used for the breeding of F₁ hybrids. The mice are housed in controlled light conditions (lights on at 03.00h, lights off at 17.00h). To induce superovulation, adult female mice are injected with 5 international units of Pregnant Mares Serum Gonadotropin (Cat. No. 4877, Sigma Chemical Company, Poole, Dorset, England) in 0.1ml of distilled water, at 15.00h followed 46 to 48 hours later by injection of 5 international units of Human Chorionic Gonadotropin (HCG) (Cat. No. CG-10, Sigma Chemical Company, Poole, Dorest, England) in 0.1 ml of distilled water. Following HCG injection, the females are housed individually with mature C57BL/6 X CBA F₁ male mice for mating. The following morning, mated female mice are identified by the presence of a vaginal plug.

Mated females are killed by cervical dislocation. All subsequent procedures are performed taking precautions to avoid bacterial and fungal contamination. Oviducts are excised and placed in M2 culture medium (Hogan, Constantini and Lacy "Manipulating the Mouse Embryo: A Laboratory Manual" Cold Spring Harbor Laboratory (1986) pp254-256). The fertilised eggs are dissected out of the ampullae of the oviducts into M2 containing 300 µg/ml hyaluronidase (Type IV-S, Cat. No. H3884, Sigma Chemical Company, Poole, Dorset, England) to release the cumulus cells surrounding the fertilised eggs.
Once the eggs are free of cumulus, they are washed free of hyaluronidase and, until required for injection, are kept at 37°C either in M2 in a humidified incubator, or in a drop (100-200 µl) of Medium No. 16 (Hogan, Constantini and Lacy "Manipulating the Mouse Embryo : A Laboratory Manual" Cold Spring Harbor Laboratory (1986) pp254-255, and 257), under mineral oil (Cat. No. 400-5. Sigma Chemical Company, Poole, Dorset, England) in an atmosphere of 95% air, 5% CO₂.

### Injection of DNA

The DNA to be injected is diluted to approximately 1.5 µg/ml in AnalaR water (Cat. No. 10292 3C, BDH Chemicals, Burnfield Avenue, Glasgow, G46 7TP, Scotland), previously sterilised by filtration through a 0.2 µm pore size filter (Cat. No. SM 16534, Sartorious, 18 Avenue Road, Belmont, Surrey SM2 6JD, England). All micropipette tips and microcentrifuge tubes used to handle the DNA and diluent are rinsed in 0.2 µm-filtered water, to remove particulate matter which could potentially block the injection pipette. The diluted DNA is centrifuged at 12000 x g for at least 15 minutes to allow any particulate matter to sediment or float; a 20 µl aliquot is removed from just below the surface and used to fill the injection pipettes.

Injection pipettes are prepared on the same day they are to be used, from 15cm long, 1.0mm outside diameter, thin wall, borosilicate glass capillaries, with filament (Cat. No. GC100TF-15; Clerk Electromedical Instruments, PO Box 8, Pangbourne, Reading, RG8 7HU, England), by using a microelectrode puller (Campden Instruments, 185 Campden Hill Road, London, England). DNA (approximately 1 µl) is introduced into the injection pipettes at the broad end; it is carried to the tip by capillary action along the filament. To prevent evaporation of water from the DNA solution, approximately 20 µl Fluorinert FC77 (Cat. No. F4758, Sigma Chemical Company, Poole, Dorset, England) is laid over the DNA solution. The filled injection pipettes are stored at 4°C until required.

The holding pipette (used to immobilise the eggs for microinjection) is prepared from 10cm long, 1.0mm outside diameter, borosilicate glass capillaries (Cat. No. GC100-10; Clark Electromedical Instruments, PO Box 8, Pangbourne, Reading, RG8 7HU, England). The glass is heated over a small flame and pulled by hand to give a 2-4cm long section with a diameter of 80-120 µm. Bends are introduced into the pipette, the glass is broken and the tip is polished using a microforge (Research Instruments, Kernick Road, Penryn, TR10 9DQ, England).

A cover slip chamber is constructed in which to micromanipulate the eggs. The base of the cover-slip chamber is a 26 x 76 x (1-1.2)mm microscope slide (Cat No. ML330-12, A and J Beveridge Limited, 5 Bonnington Road Lane, Edinburgh EH6 5BP, Scotland) siliconised with 2% dimethyldichlorosilane (Cat. No. 33164 4V, BDH Chemicals, Burnfield Avenue, Glasgow, G46 7TP, Scotland) according to the manufacturer's instructions; two glass supports (25 x 3 x 1 mm, cut from microscope slides) are fixed onto the slide with high vacuum silicone grease (Cat. No. 33135 3N, BDH Chemicals, Burnfield Avenue, Glasgow, G46 7TP, Scotland) parallel to and approximately 2mm from the long sides of the slide, half way along the length of the slide. A further two glass supports are fixed on top of the first pair, and the top surface in smeared with silicone grease. 300 µl of medium M2 are pipetted into the space between the supports, and a 22 x 22mm cover-slip (Cat. No. ML544-20, A and J Beveridge Limited, 5 Bonnington Road Lane, Edinburgh EH6 5BP, Scotland) is lowered onto the supports, a seal being formed by the grease. Dow-Corning fluid (50 cs) (Cat. No. 63006 4V, BDH Chemicals, Burnfield Avenue, Glasgow, G46 7TP, Scotland) is pipetted into the open ends of the chamber, to cover the medium.

Batches of eggs (30 to 100) are placed into a cover-slip chamber for manipulation. The chamber is mounted on the microscope (Diaphot, Nikon (UK) Ltd, Haybrooke, Telford, Shropshire, England) which has 4 x bright field, 10 x phase contrast and 40 x differential interference contrast (DIC) objectives, and 10 x eyepieces. Mechanical micromanipulators (Cat. Nos. 520 137 and 520 138, E. Leitz (Instruments) Limited, 48 Park Street, Luton, England) are mounted adjacent to the microscope and are used to control the positions of the holding and injection pipettes.

The holding pipette and DNA-containing injection pipette are mounted in modified instrument tubes (Cat. No. 520 145, E. Leitz (Instruments) Ltd, 48 Park Street, Luton, England) which are in turn mounted onto the micromanipulators via single unit (Cat. No. 520 142, E. Leitz (Instruments) Ltd, 48 Park Street, Luton, England) and double unit (Cat. No. 520 143, E. Leitz (instruments) Ltd, 48 Park Street, Luton, England) instrument holders, respectively. The instrument tubes are modified by gluing onto Clay Adams "Intramedic" adapters (2.0-3.5 mm tubing to female Luer, Cat. No. 7543D, Arnold R. Horwell Ltd, 2 Grangeway, Kilburn High Road, London, NW6 2BP, England), which are used to connect the instrument tubes to approximately 2 metres of polythene tubing (1.57 mm inside diameter, 2.9 mm outside diameter, Cat. No. F21852-0062, R.B. Radley & Co, Ltd, London Road, Sawbridgeworth, Herts, CM21 9JH, England), further "Intramedic" adapters are connected to the other ends of the polythene tubing to facilitate connection to the syringes used to control the holding and injection pipettes.

Injection is controlled using a 20ml or a 100ml glass syringe (Cat. Nos. M611/20 and M611/31, Fisons, Bishop Meadow Road, Loughborough LE11 0RG, England), the plunger of which is lightly greased with high vacuum silicone grease (Cat. No. 33135 3N, BDH Chemicals, Burnfield Avenue, Glasgow, G46 7TP, Scotland).

Holding of eggs is controlled with an Agla micrometer syringe (Cat. No. MS01, Wellcome Diagnostics, Temple Hill, Dartford DA1 5AH, England), which is fitted with a light spring around the plunger. The Agla syringe is connected via a 3-way stopcock (Cat. No. SYA-580-L), Gallenkamp, Belton Road West, Loughborough, LE11 0TR, England), to the "Intramedic" adapter, the third port of the stopcock is connected to a reservoir of Fluorinert FC77 (Cat. No. F4758, Sigma Chemical Company, Poole, Dorset, England), which fills the Agla syringe, polythene tubing, instrument tube and holding pipette.

The tip of the injection pipette is broken off against the holding pipette, to increase the tip diameter to a size which allows free passage of the DNA solution and which is small enough to allow injection without lethal damage to the eggs (≤1 µm). The flow of DNA through the pipette tip is checked by viewing under phase contrast conditions whilst pressure is applied to the injection syringe (the DNA solution will appear as a bright plume emerging from the tip of the pipette).

One by one, the fertilised eggs are picked up on the holding pipette, and one or both pronuclei brought into the same focus as the injection pipette (using the 40x objective and DIC conditions; the correction ring on the objective is adjusted for optimum resolution).
The injection pipette is inserted into one of the pronuclei, avoiding the nucleoli, pressure is applied to the injection syringe and once swelling of the pronucleus is observed, pressure is released and the injection pipette is immediately withdrawn. When pipettes block, the blockage may be cleared by application of high pressure on the injection syringe or by breaking off a further portion of the tip. If the blockage cannot be cleared, or if the pipette tip becomes dirty, the pipette is replaced.

After injection, the eggs are cultured overnight in medium No. 16 under oil in an atmosphere of 5% CO₂. Eggs which cleave to two cells during overnight culture are implanted into pseudopregnant foster mothers.

Random-bred albino (MF1, Harlan Olac Ltd, Shaw's Farm, Bicester, OX6 0TP, England) female mice are mated with vasectomized (Hogan, Costantini and Lacy, "Manipulating the Mouse Embryo: A Laboratory Manual" Cold Spring Harbour Laboratory (1986); Rafferty, "Methods in experimental embryology of the mouse", The Johns Hopkins Press, Baltimore, USA (1970)) MF1 male mice. The matings are performed one day later than those of the superovulated egg donors. MF1 females which have a detectable vaginal plug the following morning are used as foster mothers. The ideal weight of foster mothers is 25 to 30g. Each foster mother is anaesthetised by intraperitoneal injection of Hypnorm/Hypnovel (10 µl/g body weight) at 2/3 the concentration recommended by Flecknell (Veterinary Record, 113, 574) (Hypnorm: Crown Chemical Co, Ltd, Lamberhurst, Kent TN3 8DJ, England; Hypnovel: Roche Products Ltd, PO Box 8, Welwyn Garden City, Herts AL7 3AY, England) and 20 to 30 2-cell eggs are transferred into one oviduct by the method described by Hogan, Costantini and Lacy ("Manipulating the Mouse Embryo: A Laboratory Manual" Cold Spring Harbor Laboratory (1986)). As an option, to minimise bleeding from the ovarian bursa, 2 µl of 0.01% (w:v) epinephrine bitartrate (Cat. No. E4375, Sigma Chemical Company, Poole, Dorset, England) dissolved in distilled water is applied to the bursa for a few minutes before tearing it. Foster mothers are allowed to deliver their offspring naturally unless they have not done so by 19 days after egg transfer, in which case the pups are delivered by hysterectomy, and are fostered.
Following normal mouse husbandry, the pups are weaned at 3 to 4 weeks of age and housed with other mice of the same sex only.

Transgenic mice may be used for the breeding of subsequent generations of transgenic mice by standard procedures. Founder mice and Transgenice mice of subsequent generations are identified by analysis of DNA prepared from tails, as described below.

### EXAMPLE 3

### Identification of Transgenic Individuals

When the pups are at least 4 weeks of age, a biopsy of tail is taken for the preparation of DNA. The pups are anaesthetised by administration of Halothane.
Once anaesthetised, a portion of tail (1 to 2cm) is removed by cutting with a scalpel which has been heated in a Bunsen flame; the hot blade cauterises the wound and prevents bleeding.

The tail segments are digested with proteinase K 200 µg/ml (Sigma) in tail buffer [0.3 M NaAcetate (not titrated), 10mM Tris-HCl pH 7.9, 1mM EDTA pH 8.0, 1% SDS] overnight with shaking at 37°C. The following day the digests are vortexed briefly to desegregate the debris. Aliquots of digested tail are phenol/chloroform extracted once, chloroform extracted once and then DNA is recovered by precipitation with an equal volume of isopropanol.

"Tail DNA" is digested with restriction enzyme(s), and subjected to agarose gel electrophoresis. The separated DNA is then "Southern" blotted to Hybond^{™} N (Amersham) nylon membranes as described in the Amersham Handbook "Membrane transfer and detection methods" (Pl/162/86/8 published by Amersham International plc, PO Box 16, Amersham, Buckinghamshire HP7 9LL, UK).
DNA bound to the membranes is probed by hybridisation to appropriate ³²P labelled DNA sequences (eg the construct DNAs). The DNA probes are labelled with ³²P by nick-translation as described in "Molecular Cloning: A Laboratory Manual" (1982) by Maniatis, Fritsch and Sambrook, published by Cold Spring Harbor Laboratory, Box 100, Cold Spring Harbor, USA. Alternatively DNA probes are labelled using random primers by the method described by Feinberg and Vogelstein (1984) Analytical Biochemistry 137, 266-267. Briefly: The plasmid or phage is cleaved with the appropriate restriction enzymes and the desired fragment isolated from an agarose gel. The labelling reaction is carried out at room temperature by adding the following reagents in order: H₂O, 6 µl OLB*, 1.2 µl BSA, DNA (max. 25 ng), 4 µl ³²P labelled dCTP (PB10205, Amersham plc, Amersham UK), 1 µl (1 unit) Klenow Polymerise (BCL) to a final volume of 30 µl.

*OLB comprises solution A: 625 µl 2M Tris, pH 8.0 + 25 µl 5M MgC12 - 350 µl H₂O + 18 µl 2-mercaptoethanol (Sigma); solution B, 2M HEPES (Sigma), titrated to pH 6.6 with NaOH; solution C, Hexa deoxyribonucleotides (Pharmacia-LKB Biotechnology Cat. No. 27-2166-01). The labelling reaction is allowed to run overnight and then the reaction stopped by the addition of 70 µl stop solution (20 mM Nacl, 20 mM Tris pH 7.5, 2mM EDTA, 0.25% SDS, 1 µM dCTP). Incorporation is assessed by TCA precipitation and counting Cerenkov emission.

Hybridisations are carried out in sealed plastic bags by a modification of the procedure described by Church and Gilbert (1984). Proceedings of the National Academy of Sciences (USA) 81, 1991-1995. Briefly: the probe is used as a concentration of 1.5x10⁶ Cerenkov counts/ml of hybridisation buffer (HB: 0.5M sodium phosphate pH 7.2, 7% SDS, 1mM EDTA). Firstly, the membrane is prehybridised for 5 minutes in HB (15ml of buffer per 20 cm² membrane) in the plastic bag at 65°C. The probe is denatured by boiling and added to the same volume of fresh HB. The plastic bag is cut open and the prehybridisation solution drained and then the HB + probe added and the bag re-sealed. The bag and contents are incubated overnight on a rotary shaker at 60°C. After hybridisation the membrane is washed in 40 mM sodium phosphate, 1% SDS and 1mM EDTA three times for ten minutes at 65°C and then a final wash is carried out for 15-30 minutes at this temperature. Washing is monitored with a hand-held Geiger counter. The stringency of the washings may be adjusted according to the particular needs of the experiment. After the last wash the membrane is blotted dry and then placed on a dry piece of Whatman filter paper and wrapped in Saran-wrap. The membrane is exposed to x-ray film (Agfa CURIX RP-1) using an X-ray cassette at - 70°C for one or more days.

By comparison with known amounts of construct DNA treated in the same manner DNA from transgenic individuals can be identified and the number of copies of the construct DNA which have been integrated into the genome can be estimated

The same methods can be used to identify transgenic offspring of the founder transgenic individuals.

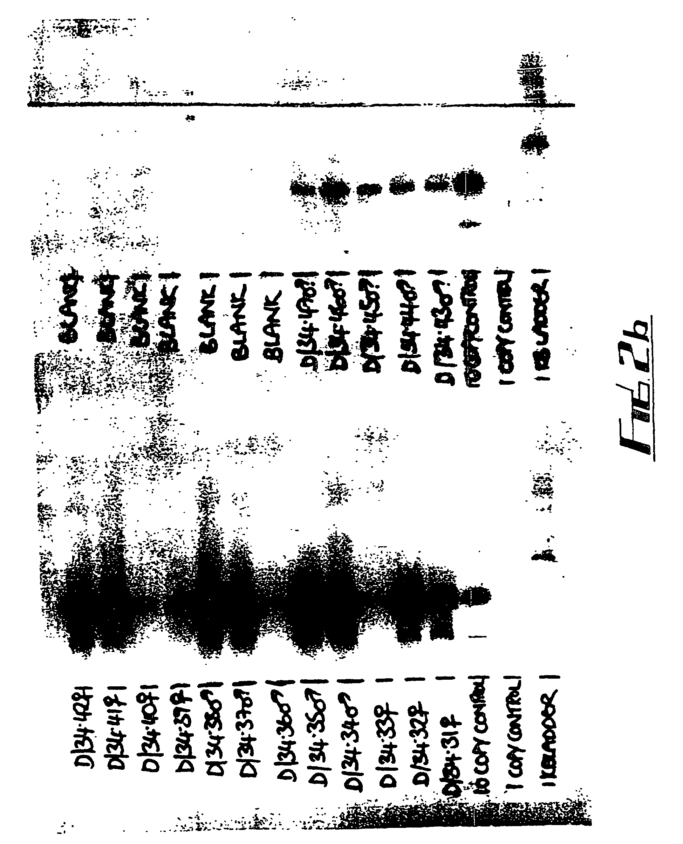

Founder individuals and transgenic mice from subsequent generations (i.e. those which have inherited the transgene) are identified by Southern Blotting as described. The ~3kb Bgl II insert derived from the β-galactosidase sequence was used as probe. Figure 2 shows the results of the Southern Blot analysis.

### EXAMPLE 4

### Breeding Transgenic Mouse Lines

From the Southern blot analysis 6 founder transgenic mice were identified (see table below) and breeding lines established:-

| Founder mice | ~Copy number | Transmission | Liver Expression |
|---|---|---|---|
| | | | (See example 5) |
| D/1 female | 40 | + | - |
| D/4 male | 4 | + | + |
| D/13 female | 39 | + | - |
| D/18 male | 50 | - | + |
| D/34 female* | 90* | + | - |
| " " " * | 4* | + | + |
| D/63 female | 30 | + | - |

| | | | |
|---|---|---|---|
| *two transgene locl identified in this founder. These segregate in the next generation to generate a high copy (~90) and a low copy (~4) line. Note that founder D/18 did not transmit the transgene sequences. | | | |

@ analysed as described in example 5.

Figure 2 shows two representative Southern blots showing the detection of the 3kb lacZ hybridising fragment in genomic digests of mouse tail DNA from generation 1 (G1) individuals from line 34 and line 4. Note the weak cross hybridising endogenous band in line 4 (evident in the negative transgenics) and the segregation of high and low copy number transgenic loci in line 34.

### EXAMPLE 5

### Detection of β-GALACTOSIDASE IN WHOLE TISSUE Abstract

The protocol described below has been adapted to analyse the β-galactosidase expression from transgenic mice and in this regard determine the activity of the P450 1A1 promoter which is effectively driving the expression of this transgene in response to induction of P450 lA1 by the carcinogenic agent 3-methylcholanthrene. All timings in this protocol are critical.

When performing this technique it is essential to incorporate a positive control into the reaction where a tissue sample which is a known expressor of β-galactosidase is treated in an identical manner to that of the test tissues. In this way it is then possible to determine, in the event of a negative result, whether the promoter is truly inactive or if the methodology was ineffective. The positive control used in these experiments was a small intestine sample which was a known high expressor of the detection enzyme.
1) The mouse under test was killed by cervical dislocation and the organs required were removed using a sterile scalpel. A small representative piece of mouse organ was fixed on to moistened cork using commercial adhesive. The tissue sample on the cork was then frozen in liquid nitrogen chilled freezing spray (Histological Supplies LTD) for approximately 30 seconds. The tissue sample was then double wrapped in parafilm, placed in a plastic bag, sealed and snap frozen in liquid nitrogen.

The remainder of the organ was wrapped in tinfoil and placed in a separate storage bag and immersed immediately in liquid nitrogen for 5 minutes and then stored at -80°C until required for experimentation work.
2) It was important that the piece of tissue for use in the β-galactosidase assay was a small flat piece suitable for gumming onto the metal chuck used in the cryostat slicing procedure.
3) The tissue sample was then placed on a piece of moistened cork covered in commercial fixing gum and frozen onto a metal chuck approximately 1.5cm in diameter using a commercial freezing spray. The chuck was immediately placed into the precooled cryostat which should be set at -22°C.

The optimal cutting temperature of the majority of tissues is -22°C. It is important that the cryostat temperature and the temperature of the tissues to be sectioned is at -22°C. At temperatures greater than this the tissues are too brittle and are prone to breakages and at temperatures greater than this artifacts may be produced.
4) The metal chuck was placed in the cryostat holder and turned until the tip of the tissues touched the front of the cutting blade. The cryostat was set to obtain small sections approximately 8 microns thickness. By turning the handle of the cryostat small sections of tissue were obtained. Sections were continually taken until a representative section of tissue was obtained. The antiroll plate was then placed against the blade and a section to be harvested was cut.
5) A slide which was coated in gelatin (0.25%) was placed against the tissue sample on the antiroll place by touching the slide on one corner of the tissue and allowing it to drop onto the remainder of the section. The tissue will bind almost immediately to the slide due to the properties of the binding substance present on the slide.
6) The slides were immediately labelled in pencil on the coloured portion of the slide the correct way up and placed in a storage rack ready for further procedure in β-galactosidase assays. Approximately six slides per tissue will be adequate to determine a representative sample for β-galactosidase activity.
7) Once the tissues are fixed onto the slides they will last indefinitely and will be stable in the freezer at -20°C or -60°C. It is extremely important to fix the slides prior to storage as they will only last for approximately one hour at room temperature unfixed.
8) Allow the slides to dry prior to fixing (approximately 30 minutes).
9) Slides were produced as described using the cryostat and the tissue was fixed in 0.5% glutaraldehyde (in PBS) for 1 day at 4°C.
10) The tissue was then incubated in PBS + 2mM MgCl₂ + 30% sucrose at 4°C overnight.
11) Wash briefly in PBS + 2mM MgCl₂ (wash solution), then for 10 minutes in fresh wash solution, this should be performed in the fridge or on ice.
12) Wash on ice for 10 minutes in detergent solution

### Detergent Solution

This can be prepared at an earlier stage and stored in the fridge. It will be stable for at least a fortnight.
PBS + per 500ml
2mM MgCl₂
0.01% sodium desoxycholate
0.02% NP40
13) 2-3 hours in X-gal solution at 37°C or even overnight.
   X-gal= 5-bromo-4-chloro-3-indoxyl-b-D-galactoside

X-gal stock = 40mg/ml in dimethylformamide Add stock to diluent to final concentration of lmg/ml while stirring in glass. It is important to do everything in glass. It is possible to make up the X-gal stock and store it in the fridge ready to add to the diluent, however it is important not to store in solution with the diluent as this will inactivate it.

### Diluent

PBS (pH 7.4) + per 500ml

| | |
|---|---|
| 5mM K₃Fe(CN)₆ | 0.82g |
| 5mM K₄Fe(CN)₆.3H₂O | 1.05g |

2mM MgCl₂
0.01% sodium desoxycholate
0.02% NP40
14) Wash 2 x 5 minutes in PBS at room temperature.
15) Wash briefly 1 x in distilled water.
16) Counterstain for 30 seconds in Orange G-1% in 2% tunsto-phosphoric acid. Alternatively alcoholic ceosin may be used for the same time period.
17) Wash 3 x 5 minutes in water.
18) The slides were then dehydrated through the following solutions by dipping for approximately 15 seconds in each and shaking.
   methylated spirits
   absolute alcohol
   absolute alcohol
   isopropanol
   histoclear
   histoclear
19) The slides were then mounted in DPX and viewed under a microscope. It was important at this stage that the slides were never permitted to dry out since this will affect the quality of the results. The slides should be continually dipped into the histoclear solution to prevent this occurring while the slides are being prepared.

### RESULTS

The expected results from the β-galacotsidase staining assay are areas of pink which uniformly stain the tissue section and small areas of blue in the tissue section which indicate areas where X-gal has been metabolised by the β-galactosidase enzyme. These results will indicate that the blue positions of the promoter under test is activated to drive the β-galactosidase enzyme.

### Analysis of liver expression

The inducibility of expression. The pAhIR1βgal transgene in the liver was examined after administration of 3-methylcholanthrene (3-mc). The inducing agent was administered IP at a dosage of 40mg/kg for 3 days and the animals were sacrificed for expression on day four. The induction in the transgenic animals was controlled by:-
a) administration of corn oil to transgenic mice,
b) the administration of 3-methylcholanthrene (3-mc) to non-transgenic littermates.

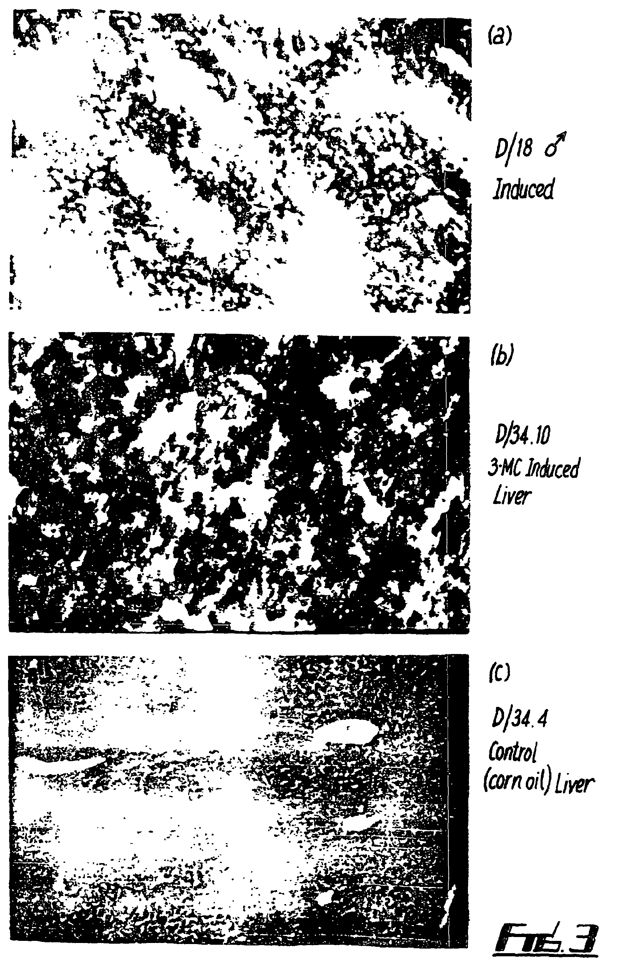

β-galactosidase expression was detected as illustrated in figure 3, three individual lines of transgenic mice were identified which showed high levels of induction in the liver. One of these (D/18) failed to transmit the transgene to the next generation and so analysis was limited to the founder male.

The levels of induction of LacZ by 3-mc were very high and importantly no basal activity of transgene expression was observed in non-induced controls (see the accompanying figure). This shows that the ~9kb rat promoter can act as an on/off switch in the liver to control gene expression.

### EXAMPLE 6

### Analysis of inducible expression in other tissues

LacZ expression was also investigated in a number of other tissues. The same procedure for sectioning and staining tissue sections was employed. 3-mc induced expression was detected in the following tissues:-
Liver
Kidney
Brain (specific regions)
Duodenum
Jejunum
Ileum
Lung
Spleen
Oesophagus
Colon
Pancreas
Adrenal Gland
Ovary

No basal activity was detected in these tissue prior to induction.

### Table 1. Regulation of the CYP1A1/β-galactosidase promoter/reporter in various mouse tissues

Experiments were carried out on 3-MC treated (40µg/g once a day for 3 days) or corn oil-treated mice carrying pAHIR1 or non-transgenic controls treated with 3-MC (+3-MC control). Various tissues were then removed and stained for β-galactosidase activity. ++++ relates to the highest intensity staining illustrated in this study in the liver. +++ through + illustrates reduced staining as compared to the liver.

| Tissue | β-Galactosidase staining intensity | | |
|---|---|---|---|
| | + 3-Mc | Corn oil | + 3-Mc Control |
| Liver | ++++ | - | - |
| Kidney | ++ | + | + |
| Duodenum | +++ | + | + |
| Jejunum | +++ | + | + |
| Ileum | +++ | + | + |
| Colon | + | - | - |
| Adrenal gland | +++ | - | - |
| Spleen | ++ | - | - |
| Oesophagus | ++ | - | - |
| Forestomach | + | - | - |
| Stomach | + | - | - |
| Pancreas | + | - | - |
| Lung | + | - | - |
| Heart | - | - | - |
| Muscle | - | - | - |
| Testes | + | - | - |
| Ovary | + | - | - |

The development of tightly regulated promoter systems are essential for many scientific and practical applications of transgenic animals. For example, in a basic research context, such models can be used to understand the cascade of metabolic processes involved in development or cell differentiation. They can also be used, in an internally controlled experiment, to understand how changes in the expression of specific genes can alter disease susceptibility. For example how the modulation of the cholesterol 7-α hydroxylase gene alters circulating cholesterol levels and as a consequence atherosclerosis. In addition the effects of dominant genes can be studied in detail and the role of dosage on their effects evaluated.

Other examples of use may include use with regulatory oncogenes to study their role in tumorigenesis, use in gene therapy and use in the direction of expression of dominant negative mutations to block inducibly the expression of endogenous genes.

The transgenic mice described herein demonstrate that the 9kb rat CYP1A1 promoter is tightly regulated in a variety of tissues. This may have very important applications in gene therapy where there is a clear requirement to modulate expression of foreign genes that have been introduced into cells. This is particularly the case for
i) cells of haematopoietic lineage - it is known that CYP1A1 genes are induced in these cells.
ii) liver
iii) lung
iv) gut

Or indeed any cell type of the body where regulation of the therapeutic gene is desirable.

For correction of genetic defects by gene therapy, this may include the gene in the homologous cell type (eg CFTR in lung epithelium or factor IX in the liver) or heterologous cell type (eg factor IX or insulin in haematopoietic cells).

In agriculture such systems have the potential to underpin the direct genetic improvement of animals by gene transfer. For example, the attempts to manipulate traits such as growth and feed efficiency in farm animals by the introduction of growth hormone genes were compromised by the chronic expression of the transgenes employed (8). By contrast, administration of recombinant growth hormone at a specific phase of development shows that if the GH transgene could be tightly regulated and induced during a specific stage of development, then such disastrous side-effects could be avoided (9).

In summary, the high level of inducibility of the CYP1A1 promoter and its activity in a wide range of tissues coupled with the low background suggest that this expression system is likely to have widespread application as a regulator of heterologous gene expression in mammals as well as other species. Furthermore, this sytem will provide a model to study the hormonal and environmental factors which regulate the expression of the CYP1A1 gene and the Ah receptor-mediated signalling pathway.

### References

1. Patient, R Curr Opionion Biotech 151-157, 1990.
2. Karin M, et al, Cell 36 371-379, 1984.
3. Wolf, C R Cancer Surveys 9, 437-474, 1990.
4. Conney, A H Pharmacol Rev, 19, 317-386, 1967.
5. Nerbert, D W, and Jones, J E Int J Biochem, 21, 243-252, 1989.
6. Sogawa K, et al, Proc Natl Acad Sol USA, 83, 8044-8048, 1986.
7. Frearing, S N, et al, Cytometry, 12, 291-301, 1991.
8. Pursel J C, et al, Science, 244, 1281-1288, 1989.
9. Etherton, T D In: Biotechnology in Growth Regulation, Butterworth pp 97-105, 1988.

## Claims

1. A recombinant genetic material which can be operably linked to a protein encoding sequence, said recombinant genetic material comprising an EcoRV-BglII restriction fragment of plasmid pAHIR1β-gal as deposited with NCIMB under Accession no. 40851, said fragment comprising an about 9kb fragment of the CYP1A1 gene said about 9kb fragment comprising the immediate 5' flanking regions, exon 1, intron 1 and part of exon II of the CYP1A1 gene.

2. A recombinant genetic material as claimed in claim 1 further comprising a protein encoding sequence to be expressed.

3. A vector incorporating recombinant genetic material as claimed in claim 1 or claim 2.

4. Host cells transfected with the recombinant genetic material as claimed in claim 1 or claim 2 or the vector as claimed in claim 3.

5. Non-human transgenic animals carrying in their genome or at least some of their cells recombinant genetic material as claimed in claim 1 or claim 2.

6. A method of manufacturing proteins or polypeptides, said method comprising the addition of an agent to a cell transformed with a construct of a recombinant genetic material as claimed in claim 1 or claim 2 to cause the induction of transcription of the protein encoding portion of said construct.

7. A method as claimed in Claim 6 wherein the agent is chosen from the group comprising PAH, TCDD, beta NF and PCBs and 3-mc.

8. The plasmid pAHIR1β-gal as deposited with NCIMB under Accession no. 40851.

9. The plasmid pAHIR1, said plasmid consisting of the plasmid pAHIR1β-gal as deposited with NCIMB under Accession no. 40851 in which the the β-gal gene is not present.

## Patentansprüche

1. Eine rekombinante genetische Substanz, die operierbar mit einer Protein codierenden Sequenz verbunden werden kann, wobei die rekombinante genetische Substanz ein EcoRV-BglII-Restriktionsfragment des pAHIR1β-gal-Plasmids wie abgelagert mit NCIMB unter Zugangsnr. 40851 beinhaltet, wobei das Fragment ein ungefähr 9kb-Fragment des CYP1 A1-Gens beinhaltet, wobei das ungefähr 9kb-Fragment die unmittelbar angrenzenden 5'-Regionen, Exon 1, Intron 1 und einen Teil von Exon II des CYP1 A1-Gens beinhaltet.

2. Rekombinante genetische Substanz gemäß Anspruch 1, die ferner eine Protein codierende Sequenz, die ausgedrückt werden soll, beinhaltet.

3. Ein Vektor, der eine rekombinante genetische Substanz gemäß Anspruch 1 oder Anspruch 2 einschließt.

4. Wirtszellen, die mit der rekombinanten genetischen Substanz gemäß Anspruch 1 oder Anspruch 2 oder dem Vektor gemäß Anspruch 3 transfiziert sind.

5. Nicht menschliche transgene Tiere, die in ihrem Genom oder zumindest in einigen ihrer Zellen rekombinante genetische Substanz gemäß Anspruch 1 oder Anspruch 2 tragen.

6. Ein Verfahren zur Herstellung von Proteinen oder Polypeptiden, wobei das Verfahren die Zugabe eines Wirkstoffs zu einer Zelle, die mit einer Konstruktion einer rekombinanten genetischen Substanz gemäß Anspruch 1 oder Anspruch 2 transformiert ist, beinhaltet, um die Induktion der Transkription des Protein codierenden Abschnitts der Konstruktion auszulösen.

7. Verfahren gemäß Anspruch 6, wobei der Wirkstoff aus der Gruppe, die PAH, TCDD, beta NF und PCBs und 3-mc beinhaltet, ausgewählt wird.

8. Das pAHIR1β-gal-Plasmid wie abgelagert mit NCIMB unter Zugangsnr. 40851.

9. Das pAHIR1-Plasmid, wobei das Plasmid aus dem pAHIR1β-gal-Plasmid wie abgelagert mit NCIMB unter Zugangsnr. 40851, in dem das β-gal-Gen nicht vorhanden ist, besteht.

## Revendications

1. Un matériel génétique recombiné qui peut être lié de façon opérationnelle à une séquence codante pour une protéine, ledit matériel génétique recombiné comportant un fragment de restriction EcoRV-Bg1II du plasmide pAHIR1β-gal tel que déposé par NCIMB sous le N° d'accès 40851, ledit fragment comportant un fragment du gène CYP1A1 d'environ 9 kb, ledit fragment d'environ 9 kb comportant les régions flanquantes 5' immédiates, exon 1, intron 1 et une partie d'exon II du gène CYP1 A1.

2. Un matériel génétique recombiné tel que revendiqué dans la revendication 1 comportant de plus une séquence codante pour une protéine devant être exprimée.

3. Un vecteur incorporant du matériel génétique recombiné tel que revendiqué dans la revendication 1 ou la revendication 2.

4. Des cellules hôtes transfectées avec le matériel génétique recombiné tel que revendiqué dans la revendication 1 ou la revendication 2 ou le vecteur tel que revendiqué dans la revendication 3.

5. Des animaux transgéniques non humains portant dans leur génome ou au moins certaines de leurs cellules du matériel génétique recombiné tel que revendiqué dans la revendication 1 ou la revendication 2.

6. Une méthode de fabrication de protéines ou de polypeptides, ladite méthode comportant l'ajout d'un agent dans une cellule transformée avec une construction d'un matériel génétique recombiné tel que revendiqué dans la revendication 1 ou la revendication 2 pour entraîner l'induction de la transcription de la portion codante pour la protéine de ladite construction.

7. Une méthode telle que revendiquée dans la revendication 6 dans laquelle l'agent est choisi dans le groupe comportant PAH, TCDD, beta NF et des PCB et 3-mc.

8. Le plasmide pAHIR1β-gal tel que déposé par NCIMB sous le N° d'accès 40851.

9. Le plasmide pAHIR1, ledit plasmide étant composé du plasmide pAHIR1β-gal tel que déposé par NCIMB sous le N° d'accès 40851 dans lequel le gène β-gal n'est pas présent.
